# EUROPEAN PATENT SPECIFICATION

(11) **EP 2 977 440 B1**
(45) Date of publication and mention of the grant of the patent: **06.03.2019**
(21) Application number: 15178146.5
(22) Date of filing: 23.07.2015
(51) Int. Cl.: C12M 1/107, C05F 17/00, C12M 1/00

(54) **PROCESS AND SYSTEM FOR THE RECYCLING IN AGRICULTURE OF THE NUTRIENTS COMING FROM THE FOOD CHAIN**
VERFAHREN UND SYSTEM ZUR WIEDERVERWERTUNG IN DER LANDWIRTSCHAFT VON NAEHRSTOFFEN AUS DER NAHRUNGSKETTE
PROCEDE ET SYSTEME POUR LE RECYCLAGE EN AGRICULTURE DES NUTRIMENTS PROVENANT DE LA CHAINE ALIMENTAIRE

(30) Priority: 25.07.2014 IT MI20141362
(43) Date of publication of application: 27.01.2016
(73) Proprietor: Neorurale S.p.A., 20124 Milano (IT)
(72) Inventor: NATTA, Francesco, 27010 GIUSSAGO (PV) (IT); NATTA, Giuseppe, 27010 GIUSSAGO (PV) (IT); DONATI, Gianni, 27010 GIUSSAGO (PV) (IT)
(74) Representative: Bianchetti Bracco Minoja S.r.l.

(56) References cited:
- EP-A1- 2 578 558
- WO-A1-2005/051852
- WO-A1-2010/098343
- CA-A1- 2 416 690
- US-A- 4 824 571
- US-A1- 2005 139 546
- US-A1- 2013 291 608

## Description

The object of the present invention is a process and a system operating continuously for the recycling of nutrients and for the preparation of fertilisers from organic substrates from waste coming from the cycle of production and consumption of foodstuffs according to claims 1 and 7. More particularly the invention relates to a new process and system for the anaerobic digestion of said organic substrates, constituted by digesters placed in series and wherein the transfer of the digestate between the digesters is performed with gas lifts actuated with compressed biogas. In each reactor a series of auxiliary gas lifts are provided which extract the digestate from various points of the base and convey it into a central conduit, performing in this way both the mixing of the digesters and an efficient extraction of the ammonia.

### State of the art

The waste deriving from the use of products of the harvest containing the nutrients, in the natural cycle and in the agricultural productions of the past, were used mainly on the land of production.

This consolidated practice was interrupted due to the urbanisation and industrialisation of the activities downstream of the agricultural cultivations, so that the waste of the food cycle is produced and disposed of in areas generally far from those in which the harvest is produced.

This problem is in addition to environmental problems, particularly felt for nitrogenated and phosphoric compounds. In fact, since the quantities removed from the cultivated soils are replaced by chemical fertilisers, the waste that derives from the cycle of production and consumption of foodstuffs, from the harvest and from human food, ends up in the environment, causing known problems of pollution of the region concerned.

Recently, however, the possibility of concentrating waste coming from the cycle of production of foodstuffs in the form of biological sludge of purification and the separation of food scraps contained in urban waste has enabled the provision of recycled nutrients in a more concentrated form and to transport them, after possible treatment, onto cultivable soils also far from the land where they are produced. In order to reduce the environmental impact due to the odours emitted by rottable waste, aerobic stabilisation processes are used, as in the production of compost from the fraction of the urban waste constituted by food scraps and in the stabilisation of the urban purification sludge and of the manure of livestock farms. This stabilisation is performed also by means of systems of anaerobic digestion, by means of which the rottable organic compounds, the source of unpleasant odours, are transformed into biogas. These processes produce materials which, if they have suitable features, often governed by law, can be intended for disposal on agricultural land.

This new approach and the wide availability of waste require the building of large-scale systems operating no longer at the single farm but on entire farming areas and even on a regional scale and the implementation of processes and technologies with high energy efficiency and yield.

Many procedures have been described, aimed mainly at obtaining biogas from the digestible components of said waste. Digestion is performed in diluted conditions and is often followed by the separation of solid materials which can be composted aerobically and of aqueous waste to be disposed of, almost never in any case for the purpose of obtaining concentrated liquid fertilisers, free from pathogens and storable in closed tanks to avoid the possible contamination thereof, without the production of other waste.

Moreover the presence of ammonia in the digester inhibits the completion of digestion, slowing down the process of anaerobic fermentation and reducing the yields.

Numerous patents relate to the anaerobic digestion of similar substrates, aimed only at production of biogas, and not at the recovery of the nutrients by means of the production of fertilisers.

The methods described to date do not completely solve the following problems:
- the need to produce a fertiliser free from pathogens and therefore fully digested and not mixed with the original waste;
- the difficulty in mixing concentrated fluids at the limit of pumpability in order to have even distribution of nutrients in the mass;
- the simultaneous need to eliminate ammonia which, in conditions of concentrated substrate, increases as digestion proceeds until anaerobic fermentation is inhibited;
- the need to supply heat economically in order to maintain large volumes at homogeneous and high temperatures, around 55°C, in order to guarantee, during thermophilic fermentation, also the pasteurisation against pathogens;
- the need to obtain as product of digestion exclusively two fluid materials which can be used as fertilisers, digestate and ammonia sulphate.

CA 1102019 discloses a combined heating and mixing system which uses a vertical conduit with jacket placed at the centre of the digester provided with means for the pumping of the suspension from below upwards.

DE 10354063 claims the digestion preferably in batches at high temperatures and in a vacuum (10-80 kPa) for the stripping of the ammonia and the absorption of the ammonia contained in the biogas exiting the digester at a lower temperature in a suspension of calcium sulphate containing possibly sulphuric acid.

EP 0563434 proposes the mixing of the sludge in fermentation with a system of pipes placed on the base of the digester in the zones wherein the same sludge and the biogas are pumped in sequence.

GB 2457681 proposes as system of mixing a moveable arm rotating on the base of the digester actuated by a flow of gas.

US 4824571 divides the base of the digester into zones provided with systems of distribution of the gas to be activated in sequence.

US 2007102352 performs the stripping of the ammonia from the product of the digester with hot air heated for example by burning biogas and treating the effluent gas with a biofilter.

Similarly WO 2010098343 heats the liquid coming from the fermenter to strip the ammonia and WO 2011018269 uses hot exhausted gases in a column where it neutralises the ammonia extracted with sulphuric acid.

US 2003/038078 performs a preliminary filtering of the waste after mixing with a recycling of the product already treated in the digester to eliminate the coarse solids and which definitely cannot be considered a hygienised fertiliser. The fluid part is treated with steam to strip the ammonia which is neutralised with acid: the digestate is recycled on the digester and is then completely transformed into biogas.

US 6299774 operates essentially in batches with an apparatus agitated mechanically and while on the one hand it is able to control the quality of the product at the end of the process, on the other hand it operates necessarily on a reduced scale.

US 6464875 is similar to US 2003/038078 yet performs filtering after digestion, extracts part of the solids and the stripping of the ammonia is performed outside of the digestion system.

CA 2416690 performs a centrifuging after the digester to give a solid fertiliser and refluent water is produced and in part used in the process and part for fertigation.

WO 2005/051852 A1 proposes performing the hygienisation of the product by means of a series of pasteurisation reactors operating in batches before performing the anaerobic fermentation in a digester. The operation in batches is required for partially emptying each reactor, varying the levels and disposing of the floating crusts.

US 2013/291608 A1 discloses a series of fermenters operating with aerobic fermentation and successive anaerobic fermentations in various working conditions, different micro-organisms and increasing temperatures. The various stages can operate on different portions of the waste separately or in combination and produce different hygienised products.

EP 2578558 A1 in the name of some of the same Applicants is aimed at the production of a digestate fertiliser and of an ammoniacal fertiliser by combining a single fermenter operating in a continuous process with an apparatus of stripping and neutralisation of the ammonia included in recycling of the digestate. The stripping of the ammonia is performed in the two versions proposed either with biogas in a closed cycle on the digester or with biogas or inert gas in closed cycle between the stripper and the neutraliser.

From the digester a high capacity of product is extracted which is functional to the stripping of the ammonia and which is recycled after adding fresh waste to the digester. While on the one hand this capacity contributes to the mixing in the digester, on the other hand it transfers with it a fraction of non-hygienised product.

As can be seen, most of these methods offer solutions aimed at the production of biogas but not at the production of fluid fertilisers having high content of dry substance in order to be able to be stored and distributed economically.

Moreover, with the exception of the batch processes, the operation on a single digester does not guarantee the non-contamination of the product with the food.

Other known processes, in addition to those described above, after the production of biogas, separate a solid phase, usable as fertiliser, from a diluted liquid, optionally disposable on the soil, yet incompatible with a substitute use of fertilisation, this use requiring the use of storage of excessive dimensions and fertilisers to be distributed in a narrow space of time.

It also has to be considered that the organic substrates are generally of faecal origin, possibly concentrated in the form of sludge of biological purification plants and therefore contain, in addition to the nutrients (N, P, K, etc.), rottable organic substances which generate unpleasant odours, possible pathogens, which cause health problems, and have variable physical state (solid, sludgy or liquid). This, together with the health and environmental problems, makes the production of homogeneous and odourless fertilisers, not contaminated by pathogens, their storage and their distribution difficult.

Examples of organic substrates comprise the manure from livestock farms, organic waste from the food and agricultural industry, biological sludge from urban purifiers and the clean wet fraction of urban waste.

In this scenario the construction of a system which allows recycling in the soil, wherefrom the harvest has been taken, of the waste of the cycle of production and consumption of foodstuffs, would solve both the problem of availability of these resources and the environmental one connected to the dispersion in the environment of excess nutrients.

### Description of the invention

A process has now been found which allows the limits and the disadvantages of known processes to be overcome and allows the recovery of nutrients and for the preparation of fertilisers from organic substrates from waste coming from the cycle of production and consumption of foodstuffs.

More particularly the digester which forms the object of the present invention allows the disadvantages of incomplete hygienisation of the product found in EP 2578558 A1 to be overcome and the system of stripping of the ammonia is simplified as described here below.

This process comprises the following steps:
a) anaerobic digestion as a continuous process in a plurality of digesters placed in series (I, II, III) of the organic substrates (1) at temperatures higher than the pasteurisation temperature, generally around 55°C, for a sufficient time for the thermophilic digestion of the same organic substrates with production of a digestate (fertiliser 1) and of biogas;
b) recirculation as a continuous process of part of the fluid present in the digesters and injection of steam to provide the heat necessary for maintaining the temperature of the same digesters and encourage the extraction of ammonia;
c) extraction as a continuous process of the ammonia by means of the sending of biogas coming from said digesters into a Venturi scrubber (V) for the neutralisation with acid to give an ammonium salt (fertiliser 2);
d) continuous extraction of biogas from a second Venturi scrubber (V1) for scrubbing for use as fuel in internal combustion engines and to provide the heat and all the energy required by the process;
e) storage of the fertiliser 1 in a tank of sufficient capacity to supply fertiliser 1 preferably in the period of fertilisation before sowing, and storage of fertiliser 2 in a tank of sufficient capacity to supply fertiliser 2 in the period of fertilisation also subsequent to sowing.
f) wherein the transfer of the digestate between the digesters is provided from the base into the ceiling of each digester (I, II, III) by means of a respective series of service gas lifts or pipes (Sa, Sb, Sc) actuated with compressed biogas and distributed along the perimeter of the digesters.

The process that is the object of the invention allows the production of two fertilisers, a mixed organic fertiliser (fertiliser 1), to be used before sowing, and a nitrogenated fertiliser (fertiliser 2), for example ammonia phosphate or sulphate, also usable with cultivations underway, starting from waste coming from the cycle of production and consumption of foodstuffs (organic substrates) and allows the performing, on a regional basis, of recycling on agricultural land of the nutrients removed by the harvest, solving the health, environmental and logistic problems sustainably and economically, being able to use for the energy requirement of the process exclusively the energy obtainable by means of digestion of said organic substrates in conditions of temperature suitable for achieving the pasteurisation of the fertilisers obtained.

The process that is the object of the present invention allows a regional system to be created, able to re-establish a sustainable farming activity, allowing the recycling of nutrients, which traditional farming performed on a local scale, also on a regional scale.

The process that forms the object of the present invention operates continuously and solves the main problems of mixing of the digester on high volumes of the same digester and in the presence of concentrated and viscous fluids. It also solves the problem of stripping of the ammonia in the presence of liquid and steam balances unfavourable for the presence of reactions in liquid phase, of the carbon dioxide and of the slowness of the phenomena of transport of matter and of heat linked to the viscosity of the fluids.

The definition of this innovative process with respect to previous ones is due to various factors originating in the pilot testing, in the performing of technological tests and in the phase of design of the industrial plant.

First of all, given the large mass of waste to be treated and the large size of the digester (15,000 m3), it made no sense to operate by parallel lines but was convenient to place at least three digesters in series (each one 5,000 m3) ensuring in this way a distribution of the residence times closer to that of a piston flow and therefore the certainty of complete decontamination of the end product.

Pumping of the dense sludge with Monyo pumps between one reactor and the other for a height of approximately 15 m presented some problems for digested sludges with high content of dry solid (over 10% and up to 17%).

The performing of technological tests with a system of pumping by means of gas lift on the sludge produced by the digestion highlighted some interesting phenomena:
- the starting pressure of the gas is approximately 15 m of water column yet the working one during pumping is less than half due to the lower density of the aerated column with consequent reduced energy consumption;
- the flow of sludge pumped increases in a proportional manner with the height of the lift with a favourable scale effect on the industrial digester;
- the sludge treated with the lift has surprisingly a measured viscosity approximately 30% lower than that of the non-treated sludge and similar to that which can be obtained by ultrasounds: the possibility is therefore offered of operating with content of dry solid in the digestate higher than 17% and higher than 21% in input, making available a major improvement in the efficiency and economy of the entire process;
- obviously the costs and the reliability of the pumping system are considerably improved.

To agitate the fermenter some patents provide mechanical circulation pumps and the invention has enabled the identification of a more favourable circulation system with gas lifts external to the fermenters actuated with compressed biogas aspirating the sludge from various points of the base of the digester and feeding it into the ceiling of the same digester so as to form a slow descending motion in the digester.

It was also found on the pilot digester that the gas lift system, as regards the stripping of the ammonia, has an efficiency comparable to more costly and complex technologies such as scraped thin films.

This efficiency improves as the height increases given the distribution of the concentration of ammonia on the industrial digester as measured on a store of large size available on site.

The biogas free from ammonia, containing little water and hot after compression, improves the stripping of the ammonia further.

The pumping efficiency also improves almost proportionally with the height of the digester and therefore of the lift.

Each digester in this way is provided with its system of stripping the ammonia as it forms.

All these observations have allowed, with respect to EP 2578558 A1, elimination of the apparatus of stripping of the ammonia, replacing it with the simpler pipes of the gas lifts, reduction of the recirculated flow of sludge on the digester to that which is strictly necessary for the mixing of the food waste and also reduction of the gas flow necessary for the stripping of the ammonia.

The biogas produced is fed to internal combustion engines for the production of electrical energy and heat.

A further advantage was identified in the possibility of use of the heat of the compressed gas and of the steam recovered from the residual heat of the internal combustion engines injected into the transfer lifts for the heating of the digesters to the optimal temperature of 55°C. The steam injected into the lift also has an enormous effect on the stripping of the ammonia thanks to the induced vibrations which improve transfers of material and above all to the local raising of the temperature which encourages the balances in the gaseous phase.

The compressed biogas is in fact hot over 100°C and with reduced humidity and operates effectively also through the service lifts.

The advantages already reported, which simplify and improve the efficiency and the economical nature of the process and of the system, are in addition to the flexibility of management obtained by means of the modulation of the compressed service biogas for its use in a continuous process or according to programmed cycles or mainly in the digesters where it is more necessary.

It is also possible to reverse occasionally the flow of the gas and of the digestate in the digesters by means of appropriate cut-off valves on the series of service gas lifts.

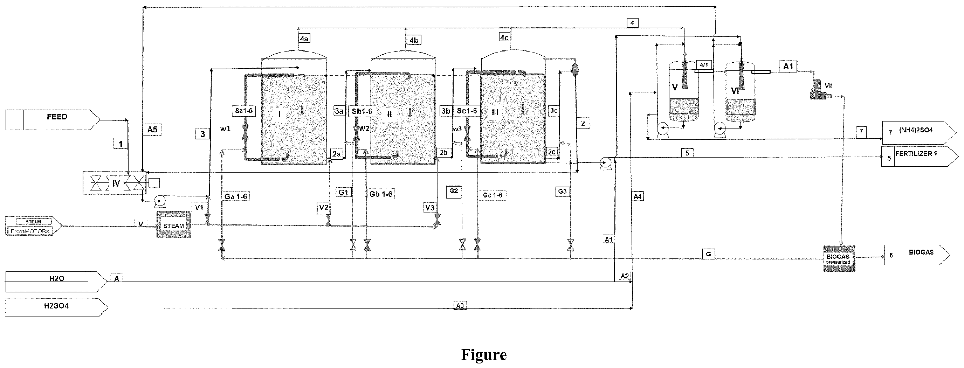

The invention has enabled development of the diagram shown in the drawing on the basis whereof the industrial system was designed and built, which comprises:
a) a plurality of digesters placed in series (I, II, III), apt to perform an anaerobic digestion as a continuous process of the organic substrates (1) at temperatures higher than the pasteurisation temperature, generally around 55°C, for a sufficient time for the thermophilic digestion of the same organic substrates with production of a digestate (fertiliser 1) and of biogas;
b) means for the recirculation as a continuous process of part of the fluid present in the digesters and means for the injection of steam to provide the heat necessary for maintaining the temperature of the same digesters and encourage the extraction of ammonia;
c) a first Venturi scrubber (V) fed with biogas coming from said digesters for the neutralisation of the ammonia with acid to give an ammonium salt (fertiliser 2);
d) a second Venturi scrubber (V1) for the scrubbing with water of the biogas exiting the first Venturi scrubber (V) and at least one compressor (VII) for compressing said biogas for use as fuel in internal combustion engines and to provide the heat and all the energy required by the process;
e) a tank for storage of fertiliser 1 of sufficient capacity to supply fertiliser 1 preferably in the period of fertilisation before sowing, and a tank for storage of fertiliser 2 of sufficient capacity to supply fertiliser 2 in the period of fertilisation also subsequent to sowing
f) a series of service gas lifts or pipes (Sa, Sb, Sc) distributed along the perimeter of the digesters for the transfer of the digestate from the base into the ceiling of each respective digester (I, II, III). According to the invention, the process provides for:
   - three digesters in series whereof the first I fed via the line 3 with pump by the mixer of the concentrated feed 1 with the recycling of the digestate 7 and process water A5, the second II and the third III via the gas lifts corresponding to the lines 3a and 3b;
   - in the lines 3, 3a and 3b the steam V1, V2, V3 is injected for the heating of the digesters and the improvement of the stripping of the ammonia;
   - on each digester a series of service gas lifts or pipes Sa, Sb, Sc are provided, distributed along the perimeter of the digester and actuated with hot and compressed biogas which take the digestate from the base and transport it into the ceiling of the digester. These pipes are intercepted by valves so as to allow temporarily and according to needs the reversal of the flows of gas and digestate in the digester;
   - the biogas exiting from the ceiling of the digesters 4a, 4b, 4c is sent via the line 4 to a Venturi scrubber V where the ammonia is neutralised with sulphuric acid A3 diluted with water A2 to give the flow A4 and subsequently scrubbed in another Venturi scrubber with water 4/2; the water exiting with the flow A5 is used to dilute the feed 1;
   - the scrubbed biogas 4/3 is compressed and in part sent to the internal combustion engines with the flow 6 and the most part G used in the lifts of the digesters for the transfers of digestate, for the stripping of the ammonia and for agitating;
   - the ammonia sulphate 7 produced in the Venturi scrubber is sent to the storage of the fertiliser 2;
   - the digestate from the last digester is for the most part transferred via line 2 with lift or with pump to dilute the feed 1 and in part sent via the line 5 to the storage of the fertiliser 1.

The tanks for storage of the fertilisers produced are of a size functional to the quantity and their period of use: fertiliser 1 for the period before sowing and fertiliser 2 for the period after sowing.

### Example 1

The example relates to the process described and its use in a centre serving agricultural land. The centre deals overall with the following organic substrates, coming from the cycle of production and consumption of foodstuffs:
- 12,000 tonnes/year of poultry droppings
- 20,000 tonnes/year of thickened biological sludge of a food and agricultural industrial concern
- 50,000 tonnes/year of filter pressed urban sludge
to obtain:
- 100,000 tonnes/year of organic mixed fertiliser 1 in aqueous dispersion
- 5,850 tonnes/year of fertiliser 2 consisting of a 20% ammonia sulphate solution.

The organic substrates described above and which constitute the feed 1, with a flow rate of approximately 20 tonnes/hour, are roughly mixed one with the other in IV, with the recycling 2 of the digestate and with the process water A5.

The product homogenised in this way is fed to the digester I together with the heating steam V1 with an hourly flow rate of approximately 60 mc/hour and subsequently passes in a cascade to the digesters II and III by means of the gas lifts 3a and 3b, also heated with steam.

In the cascade of fermenters a temperature profile around 55°C is formed and in any case variable by measuring the flows of steam V1, V2, V3 according to process needs.

The volume of the single digester is 5,000 m3 (working 4,500 m3) and each of them is provided with a series of six service gas lifts Sa, Sb, Sc for the mixing and the stripping of the ammonia. The recycled flow of digestate of each of the service lifts is similar to that of the transfer lifts and the time of mixing approximately 10-12 h, in line with the needs of a process which has an overall residence time of 24 days.

The eighteen gas lifts Sa, Sb, Sc are provided with valves w1, w2, w3 in the case wherein a temporary reversal is to be performed of the flows in the digesters, for example for a local block of one thereof or to shift the base of the digester.

The biogas which exits from the digesters is in slight overpressure and passes into two Venturi scrubbers where, without additional load losses, it undergoes the neutralisation of the ammonia and is scrubbed with water before being aspirated by the compressor VII.

The compressor VII can compress the biogas up to 2.5 bar but in normal working conditions it operates at around 1.5 bar and mostly recycles the biogas to the process and in part to the cogeneration plant.

Two compressors are installed on the plant in parallel for ensuring operations and for possible production increases.

The cogeneration plant has an electrical power of 1,000 kWe and an availability of heat with the fumes and the cooling water of 1,600 kWt.

The fertiliser 1 produced is fed to the storage tank, having capacity of 50,000 mc with floating roof and similarly fertiliser 2 in a 6,000 mc tank.

The two tanks of fertiliser 1 and the tank of fertiliser 2 are connected with the station of loading of the means for the transport of the fertilisers. Here below the methods are given of dimensioning of the centre serving an area of agricultural land which comprises five municipalities in a territorial region represented by the provinces of Milan and Pavia where organic substrates, coming from said territorial region, are collected.

The arable surface served by the centre is 10,120 hectares with the following crops: Rice: 7,800 ha, Corn: 1,220 ha, Wheat: 563 ha, Soya: 211 ha, Other: 326 ha.

The following livestock farms are present in the area: No. of heads of poultry: 39,277, No. of heads of cattle: 2,674, No. of heads of pigs: 5,188.

The net requirement of nutrients required by the centre can be estimated as the difference between the quantity of the various elements required by the crops present in the area and that available in the area and in the particular case: N 1471 - 422 = 1049 tonnes/year, P2O5 601-84 = 517 tonnes/year, K2O 703-113 = 590 tonnes/year.

As can be noted, most of the nutrients are dispersed in the food cycle and have to be recovered far from the farming from production and processing systems or zones where moreover their alienation represents a problem.

The availability of centres serving areas of agricultural land distributed in the area allows a virtuous circle to be closed again which had been interrupted with industrialisation no longer on the scale of the single property but on a regional scale with enormous economic and environmental advantages.

The invention obviously also comprises possible variations on the flow diagrams of the embodiments described above, within the reach of persons skilled in the art without thereby departing from the spirit and scopes of the invention.

The process described can advantageously be implemented in centres for the recovery of the nutrients removed by the harvesting of agricultural land, in which during the year the waste coming from the cycle of production and consumption of the foodstuffs (organic substrates) is collected. Two fertilisers are thus obtained which are stored at the end of their use in the period of fertilisation: a first fertiliser containing also stable organic substance can be used preferably before sowing while a second fertiliser of the inorganic type can also be used after sowing.

## Claims

1. A process for the recovery of nutrients and for the preparation of fertilisers from organic substrates from waste coming from the cycle of production and consumption of foodstuffs comprising the following steps:
a) anaerobic digestion as a continuous process in a plurality of digesters placed in series (I, II, III) of the organic substrates (1) at temperatures higher than the pasteurisation temperature, generally around 55°C, for a sufficient time for the thermophilic digestion of the same organic substrates with production of a digestate (fertiliser 1) and of biogas;
b) recirculation as a continuous process of part of the fluid present in the digesters and injection of steam to provide the heat necessary for maintaining the temperature of the same digesters and encourage the extraction of ammonia;
c) extraction as a continuous process of the ammonia by means of the sending of biogas coming from said digesters into a Venturi scrubber (V) for the neutralisation with acid to give an ammonium salt (fertiliser 2);
d) continuous extraction of biogas from a second Venturi scrubber (V1) for scrubbing for use as fuel in internal combustion engines and to provide the heat and all the energy required by the process;
e) storage of fertiliser 1 in a tank of sufficient capacity to supply fertiliser 1 preferably in the period of fertilisation before sowing, and storage of fertiliser 2 in a tank of sufficient capacity to supply fertiliser 2 in the period of fertilisation also subsequent to sowing;
f) wherein the transfer of the digestate between the digesters is provided from the base into the ceiling of each digester (I, II, III) by means of a respective series of service gas lifts or pipes (Sa, Sb, Sc) actuated with compressed biogas and distributed along the perimeter of the digesters.

2. A process according to claim 1, wherein the first (I) of said series of digesters is fed with said concentrated organic substrates (1) and with the recycled digestate and process water coming out of said second Venturi scrubber (V1), while the successive digesters (II, III) are fed via gas lift.

3. A process according to claim 1 or 2, **characterised in that** the feeding is provided into said series of service pipes or gas lifts (Sa, Sb, Sc) of part of the hot biogas exiting said Venturi scrubber (V1) previously subjected to compression.

4. A process according to any one of the preceding claims, wherein said service gas lifts or pipes (Sa, Sb, Sc) are intercepted by valves (W1, W2, W3) so as to allow temporarily and according to needs the reversal of the flows of gas and digestate in each digester, for example for a local block of one thereof or for shifting the base of the digester.

5. A process according to claim 1 and one or more of the preceding claims, wherein the digestate from the last digester (III) is for the most part transferred by means of a line (2) with lift or with pump to dilute said concentrated organic substrates (1) and in part sent via a line (5) to the storage of the fertiliser 1.

6. A process according to any one of the preceding claims wherein part of the biogas is used as fuel in a plant of cogeneration of electrical energy where residual heat is available with the cooling water and the exhaust fumes of the engines.

7. System for the recovery of nutrients and for the preparation of fertilisers from organic substrates from waste coming from the cycle of production and consumption of foodstuffs comprising:
a) a plurality of digesters placed in series (I, II, III) apt to perform an anaerobic digestion as a continuous process of the organic substrates (1) at temperatures higher than the pasteurisation temperature, generally around 55°C, for a sufficient time for the thermophilic digestion of the same organic substrates with production of a digestate (fertiliser 1) and of biogas;
b) means for the recirculation as a continuous process of part of the fluid present in the digesters and means for the injection of steam to provide the heat necessary for maintaining the temperature of the same digesters and encourage the extraction of ammonia;
c) a first Venturi scrubber (V) fed with biogas coming from said digesters for the neutralisation of the ammonia with acid to give an ammonium salt (fertiliser 2);
d) a second Venturi scrubber (V1) for the scrubbing with water of the biogas exiting the first Venturi scrubber (V) and at least one compressor (VII) for compressing said biogas for use as fuel in internal combustion engines and to provide the heat and all the energy required by the process;
e) a tank for storage of fertiliser 1 of sufficient capacity to supply fertiliser 1 preferably in the period of fertilisation before sowing, and a tank for storage of fertiliser 2 of sufficient capacity to supply fertiliser 2 in the period of fertilisation also subsequent to sowing;
f) a series of service gas lifts or pipes (Sa, Sb, Sc) distributed along the perimeter of the digesters for the transfer of the digestate from the base into the ceiling of each respective digester (I, II, III).

## Patentansprüche

1. Verfahren zur Gewinnung von Nährstoffen und zur Herstellung von Düngemitteln aus organischen Substraten aus Abfall, der aus dem Produktions- und Verbrauchszyklus von Nahrungsmitteln kommt, umfassend die folgenden Schritte:
a) anaerober Verdau als ein kontinuierliches Verfahren in einer Vielzahl an Verdauanlagen, die in Serie (I, II, III) angeordnet sind, der organischen Substrate (1) bei Temperaturen höher als der Pasteurisationstemperatur, im Allgemeinen um 55°C, für eine ausreichende Zeit für den thermophilen Verdau derselben organischen Substrate unter Herstellung eines Verdaurückstands (Düngemittel 1) und von Biogas;
b) Rezirkulation als ein kontinuierliches Verfahren eines Teils des Fluids, das in den Verdauanlagen vorliegt, und Einspritzen von Dampf, um die Wärme bereitzustellen, die notwendig ist, um die Temperatur derselben Verdauanlagen beizubehalten und die Extraktion von Ammoniak zu fördern;
c) Extraktion als ein kontinuierliches Verfahren des Ammoniaks durch Schicken des aus den Verdauanlagen kommenden Biogases in einen Venturi-Wäscher (V) für die Neutralisation mit Säure, um ein Ammoniumsalz (Düngemittel 2) zu ergeben;
d) kontinuierliche Extraktion von Biogas aus einem zweiten Venturi-Wäscher (V1) zum Reinigen, zur Verwendung als Brennstoff in Verbrennungsmotoren, und um die Wärme und die gesamte Energie, die von dem Verfahren benötigt werden, bereitzustellen;
e) Speichern des Düngemittels 1 in einen Behälter ausreichender Kapazität, um das Düngemittel 1 vorzugsweise im Zeitraum der Düngung vor dem Aussäen zur Verfügung zu stellen, und Speichern des Düngemittels 2 in einem Behälter ausreichender Kapazität, um das Düngemittel 2 im Zeitraum der Düngung auch nach dem Aussäen zur Verfügung zu stellen;
f) wobei die Übertragung des Verdaurückstands zwischen den Verdauanlagen vom Boden in die Decke jeder Verdauanlage (I, II, III) mittels einer entsprechenden Serie an Servicegaslifts oder -leitungen (Sa, Sb, Sc), die mit komprimiertem Biogas betrieben werden und entlang des Umfangs der Verdauanlagen verteilt sind, bereitgestellt wird.

2. Verfahren gemäß Anspruch 1, wobei die erste (I) der Serie an Verdauanlagen mit den konzentrierten organischen Substraten (1) und mit dem recycelten Verdaurückstand und Verfahrenswasser, herauskommend aus dem zweiten Venturi-Wäscher (V1), beschickt wird, während die nachfolgenden Verdauanlagen (II, III) über einen Gaslift beschickt werden.

3. Verfahren gemäß Anspruch 1 oder 2, **dadurch gekennzeichnet, dass** das Beschicken bereitgestellt wird in die Serie an Serviceleitungen oder -gaslifts (Sa, Sb, Sc) eines Teils des heißen Biogases, das aus dem Venturi-Wäscher (V1) austritt, das vorher Kompression unterworfen wurde.

4. Verfahren gemäß einem der vorausgehenden Ansprüche, wobei die Servicegaslifts oder -leitungen (Sa, Sb, Sc) durch Ventile (W1, W2, W3) unterbrochen sind, um so temporär und gemäß dem Bedarf die Umkehr der Ströme von Gas und Verdaurückstand in jeder Verdauanlage zu erlauben, zum Beispiel für eine lokale Blockierung einer davon oder für das Verschieben des Bodens der Verdauanlage.

5. Verfahren gemäß Anspruch 1 und einem oder mehreren der vorangehenden Ansprüche, wobei der Verdaurückstand aus der letzten Verdauanlage (III) größtenteils mittels einer Leitung (2) mit einem Lift oder einer Pumpe übertragen wird, um die konzentrierten organischen Substrate (1) zu verdünnen, und teilweise über eine Leitung (5) zu der Speicherung des Düngemittels 1 geschickt wird.

6. Verfahren gemäß einem der vorangehenden Ansprüche, wobei ein Teil des Biogases als Brennstoff in einer Kraft-Wärme-Kopplungs-Anlage elektrischer Energie verwendet wird, wo Restwärme mit dem Kühlwasser und den Abgasen der Motoren verfügbar ist.

7. System für die Gewinnung von Nährstoffen und für die Herstellung von Düngemitteln aus organischen Substraten aus Abfall, der aus dem Produktions- und Verbrauchszyklus von Nahrungsmitteln kommt, umfassend:
a) eine Vielzahl an Verdauanlagen, die in Serie (I, II, III) angeordnet sind, geeignet, um einen anaeroben Verdau als ein kontinuierliches Verfahren der organischen Substrate (1) bei Temperaturen höher als der Pasteurisationstemperatur, im Allgemeinen um 55°C, für eine ausreichende Zeit für den thermophilen Verdau derselben organischen Substrate durchzuführen, unter Herstellung eines Verdaurückstands (Düngemittel 1) und von Biogas;
b) Mittel für die Rezirkulation als ein kontinuierliches Verfahren eines Teils des Fluids, das in den Verdauanlagen vorliegt, und Mittel für das Einspritzen von Dampf, um die Wärme bereitzustellen, die notwendig ist, um die Temperatur derselben Verdauanlagen beizubehalten und die Extraktion von Ammoniak zu fördern;
c) einen ersten Venturi-Wäscher (V), der mit Biogas, das aus den Verdauanlagen kommt, beschickt wird, für die Neutralisation des Ammoniaks mit Säure, um ein Ammoniumsalz (Düngemittel 2) zu ergeben;
d) einen zweiten Venturi-Wäscher (V1) für das Reinigen mit Wasser des Biogases, das aus dem ersten Venturi-Wäscher (V) austritt, und wenigstens einen Kompressor (VII) für das Komprimieren des Biogases, zur Verwendung als Brennstoff in Verbrennungsmotoren und um die Wärme und die gesamte Energie, die von dem Verfahren benötigt werden, bereitzustellen;
e) einen Behälter zum Speichern des Düngemittels 1 ausreichender Kapazität, um das Düngemittel 1 vorzugsweise im Zeitraum der Düngung vor dem Aussäen zur Verfügung zu stellen, und einen Behälter zum Speichern eines Düngemittels 2 ausreichender Kapazität, um das Düngemittel 2 im Zeitraum der Düngung auch nach dem Aussäen zur Verfügung zu stellen;
f) eine Serie an Servicegaslifts oder -leitungen (Sa, Sb, Sc), die entlang des Umfangs der Verdauanlagen verteilt sind, für die Übertragung des Verdaurückstands vom Boden in die Decke jeder entsprechenden Verdauanlage (I, II, III).

## Revendications

1. Procédé de récupération d'éléments nutritifs et de préparation de fertilisants à partir de substrats organiques de déchets provenant du cycle de production et de consommation d'aliments comprenant les étapes suivantes :
a) la digestion anaérobie en un processus continu dans une pluralité de digesteurs placés en série (I, II, III) des substrats organiques (1) à des températures supérieures à la température de pasteurisation, généralement autour de 55 °C, durant un temps suffisant pour la digestion thermophile des mêmes substrats organiques avec la production d'un digestat (fertilisant 1) et de biogaz ;
b) la recirculation en un processus continu d'une partie du fluide présent dans les digesteurs et l'injection de vapeur pour fournir la chaleur nécessaire afin de maintenir la température des mêmes digesteurs et d'encourager l'extraction de l'ammoniaque ;
c) l'extraction en un processus continu de l'ammoniaque au moyen de l'envoi du biogaz desdits digesteurs dans un épurateur de Venturi (V) pour la neutralisation avec de l'acide pour donner un sel d'ammonium (fertilisant 2) ;
d) extraction continue du biogaz d'un second épurateur de Venturi (V1) pour l'épuration pour une utilisation comme carburant dans des moteurs à combustion interne et pour fournir la chaleur et toute l'énergie requise par le processus ;
e) le stockage du fertilisant 1 dans un réservoir de capacité suffisante pour fournir le fertilisant 1 de préférence dans la période de fertilisation avant les semailles, et le stockage du fertilisant 2 dans un réservoir de capacité suffisante pour fournir le fertilisant 2 dans la période de fertilisation aussi après les semailles ;
f) où le transfert du digestat entre les digesteurs est fourni à partir de la base dans le plafond de chaque digesteur (I, II, III) au moyen d'une série respective d'appareils d'ascension de gaz de service ou de tuyaux (Sa, Sb, Sc) actionnés avec du biogaz comprimé et distribués le long des périmètres des digesteurs.

2. Procédé selon la revendication 1, dans lequel le premier (I) de la dite série de digesteurs est alimenté avec lesdits substrats organiques concentrés (1) et avec le digestat recyclé et l'eau du processus provenant dudit second épurateur de venturi (V1), alors que les digesteurs successifs (II, III) sont alimentés via un appareil d'ascension de gaz.

3. Procédé selon la revendication 1 ou 2, **caractérisé en ce que** l'alimentation est fournie dans lesdites séries de tuyaux de service ou de levages de gaz (Sa, Sb, Sc) d'une partie du biogaz chaud sortant dudit épurateur de Venturi (V1) préalablement soumis à une compression.

4. Procédé selon l'une quelconque des revendications précédentes, dans lequel lesdits levages de gaz de service ou tuyaux (Sa, Sb, Sc) sont interceptés par des vannes (W1, W2, W3) pour permettre temporairement et selon les besoins l'inversion des écoulements de gaz et de digestat dans chaque digesteur, par exemple pour un bloc local d'un de ceux-ci ou pour déplacer la base du digesteur.

5. Procédé selon la revendication 1 et une ou plusieurs des revendications précédentes, dans lequel le digestat du dernier digesteur (III) est pour la plus grande partie transféré au moyen d'une ligne (2) avec un appareil d'ascension ou avec une pompe pour diluer lesdits substrats organiques concentrés (1) et en envoyer en partie via une ligne (5) au stockage du fertilisant 1.

6. Procédé selon l'une quelconque des revendications précédentes dans lequel une partie du biogaz est utilisé comme carburant dans une usine de cogénération d'énergie électrique où de la chaleur résiduelle est disponible avec l'eau de refroidissement et les fumées d'échappement des moteurs.

7. Système de récupération d'éléments nutritifs et de préparation de fertilisants à partir de substrats organiques de déchets provenant du cycle de production et de consommation d'aliments comprenant :
a) une pluralité de digesteurs placés en série (I, II, III) aptes à effectuer la digestion anaérobie en un processus continu des substrats organiques (1) à des températures supérieures à la température de pasteurisation, généralement autour de 55 °C, durant un temps suffisant pour la digestion thermophile des mêmes substrats organiques avec la production d'un digestat (fertilisant 1) et de biogaz ;
b) un moyen pour la recirculation en un processus continu d'une partie du fluide présent dans les digesteurs et un moyen pour l'injection de vapeur pour fournir la chaleur nécessaire afin de maintenir la température des mêmes digesteurs et d'encourager l'extraction de l'ammoniaque ;
c) un premier épurateur de venturi (V) alimenté avec du biogaz provenant desdits digesteurs pour la neutralisation de l'ammoniaque avec de l'acide pour donner un sel d'ammonium (fertilisant 2) ;
d) un second épurateur de Venturi (V1) pour l'épuration avec de l'eau du biogaz sortant du premier épurateur de Venturi (V) et au moins un compresseur (VII) pour comprimer ledit biogaz pour une utilisation comme carburant dans des moteurs à combustion interne et pour fournir la chaleur et toute l'énergie requise par le processus ;
e) un réservoir pour le stockage du fertilisant 1 de capacité suffisante pour fournir le fertilisant 1 de préférence dans la période de fertilisation avant les semailles, et un réservoir pour le stockage du fertilisant 2 de capacité suffisante pour fournir le fertilisant 2 dans la période de fertilisation aussi après les semailles ;
f) une série de levage de gaz de service ou de tuyaux (Sa, Sb, Sc) distribués le long des périmètres des digesteurs pour le transfert du digestat à partir de la base dans le plafond de chaque digesteur respectif (I, II, III).
